# FASCICULE DE BREVET EUROPEEN

(11) **EP 1 990 404 B1**
(45) Date de publication et mention de la délivrance du brevet: **19.03.2014**
(21) Numéro de dépôt: 08008754.7
(22) Date de dépôt: 09.05.2008
(51) Int. Cl.: C12N 1/14, A01N 63/00, C12R 1/885

(54) **Souche de trichoderma atroviride, procédé d'isolement d'une telle souche, procédé d'obtention d'un produit à base d'une telle souche et utilisation d'une telle souche**
Stamm des Pilzes Trichoderma atroviride, Isolationsverfahren eines solchen Stamms, Verfahren zur Herstellung eines Produkts auf der Basis dieses Stamms und Verwendung dieses Stamms
Trichoderma atroviride strain, method of isolating such a strain, method of obtaining a product based on such a strain and use of such a strain

(30) Priorité: 09.05.2007 FR 0703293
(43) Date de publication de la demande: 12.11.2008
(73) Titulaire: AGROLOR Centre d'Affaires "Le Cahn", 54100 Nancy (FR)
(72) Inventeur: Dhuicq, Laurent, 54130 Saint-Max (FR)
(74) Mandataire: Poupon, Michel

(56) Documents cités:
- EP-A- 1 279 335
- JP-A- 11 225 745
- ELMER P A G ET AL: "Biosuppression of Botrytis cinerea in grapes" PLANT PATHOLOGY (OXFORD), vol. 55, no. 2, avril 2006 (2006-04), pages 155-177, XP002462820 ISSN: 0032-0862
- SCHUBERT MARK: 'In vitro und ad planta Studien zum Einsatz von Trichoderma-Arten für die Biologische Kontrolle Holz abbauender Pilze an Bäumen', [en ligne] 2006, FREIBURG IM BREISGAU, Extrait de l'Internet: <URL:http://webdoc.sub.gwdg.de/ebook/dissts /Freiburg/Schubert2007.pdf>

## Description

La présente invention concerne l'utilisation de l'espèce Trichoderma atroviride contre les maladies des plantes, et plus particulièrement une nouvelle souche de *Trichoderma atroviride,* appelée AGR2, et son utilisation comme produit phytosanitaire destiné à la protection des cultures contre les maladies cryptogamiques, notamment pour la vigne.

La nouvelle souche de *Trichoderma atroviride AGR2* a été isolée d'un sol cultivé français et déposée auprès de la Collection Nationale des Cultures de Micro-organismes (CNCM) de l'Institut Pasteur sous le numéro I-2738, le 19 octobre 2001.

Les champignons du genre *Trichoderma* sont généralement utilisés comme produit de base pour de nombreuses préparations phytosanitaires, destinées au traitement des maladies cryptogamiques.

Ainsi, le brevet FR 2 841 436 décrit une composition de micro-organismes utilisée pour combattre certaines maladies cryptogamiques touchant la vigne, telles que l'esca ou l'eutypiose. Cette composition est constituée d'un mélange d'une bactérie, d'une levure et d'un champignon, notamment une souche de *Trichoderma.*

Le brevet FR 2 8694 832 décrit une souche particulière de *Trichoderma harzanium* constituant la base d'un produit phytosanitaire destiné à la lutte biologique de maladies cryptogamiques du bois de vigne, telles que l'esca ou l'eutypiose.

Le document Elmer P A G ET AL. : « Biosuppression of Botrytis cinerea in grapes » PLANT PATHOLOGY (OXFORD), vol. 55, no. 2, avril 2006, pages 155-177, décrit l'utilisation de Trichoderma atroviride LC52 dans la lutte contre les maladies des plantes, en particulier contre Botrytis cinerea.

Le document JP 11 225745 décrit l'utilisation de Trichoderma atroviride SKT-1, SKT-2 et SKT-3 dans la lutte contre les maladies des plantes.

Le document EP1 279 335 décrit l'utilisation de l'espèce Trichoderma atroviride dans la lutte contre les maldies des plantes, en tant que produit phytosanitaire par pulvérisation, en tant que produit phytosanitaire constitué de ce seul micro-organisme et en tant que produit destiné à la simulation racinaire, de la germiçnation et de la croissance des plantes.

Le document Schubert Mark « in vitro und ad planta Studien zum Einsatz von Trichoderma-Arten für die Biologische Kontrolle Holz abbauender Pilze an Bäumen" 2006, Freiburg im Breisgau Extrait de l'Internet: URL:http://webdoc.sub.gwdg.de/ebook/dissts/Freiburg/Schubert2007.pdf divulgue une étude sur l'emploi de diverses souches de Trichoderma pour le contrôle biologique de champigons linophages sur des arbres.

La présente invention propose l'espèce Trichoderma atroviride ainsi qu'une nouvelle souche de *Trichoderma atroviride* et leur utilisation, seule ou dans un produit phytosanitaire, pour la protection des cultures contre les maladies cryptogamiques, notamment pour la vigne contre les maladies du bois :
- l'Esca, due à un complexe fongique, composé de deux catégories de champignons, les champignons pionniers (Eutypa lata, Phaemoniella *chlamydospora, Phaeoacremonium aleophilum*) et les champignons secondaires (*Fomitiporia punctata, Stereum hirsutum),*
- le Black Dead Arm, dû à *Botryosphaeria dothidea, B. stevensii, Botryophaeria spp,*
- l'Eutypiose causée par un seul champignon, *Eutypa tata,* pénétrant dans le bois à partir des plaies de taille, causant un dépérissement du bois,
- et l'Excoriose dûe à *Phomopsis viticola.*

Selon l'invention l'espèce *Trichoderma atroviride* en général ainsi que la nouvelle souche AGR2 en particulier permettent également la lutte contre les maladies cryptogamiques pour toutes les cultures, notamment :
- le Botrytis dû aux *Botrytis cinérea, B. aili,*
- le Sclérotinia dû aux *S. sclerotiorum, S. minor, S. cepivorum,*
- la Fusariose dû aux *Microdocchium nivale, F. oxysporum,*
- l'Armillaire dû à *Armillaria mella.*

A cet effet, la présente invention a pour objet l'utilisation de l'espèce *Trichoderma atroviride* dans la lutte contre les maladies des plantes.
Elle concerne également une souche de *Trichoderma atroviride* caractérisée en ce qu'il s'agit de la souche *Trichoderma atroviride AGR2* déposée à la Collection Nationale de Cultures de Micro-organismes (CNCM) de l'Institut Pasteur sous le numéro I-2738.

La souche *Trichoderma atroviride* AGR2 selon l'invention a été identifiée au moyen de diverses méthodes.
L'observation microscopique de la morphologie, de la mesure des éléments conidiogènes et des conidies globuleuses non ornementées, ainsi qu'une odeur caractéristique ont conclu à un champignon de l'espèce *Trichoderma atroviride.*
L'identification de la souche a été affinée par une analyse moléculaire basée sur le séquençage de la région ITS1-ITS2 des ARNr (acide ribonucléique ribosomal) 18S et 25S. Une comparaison des homologies de ces régions avec la séquence de la souche de référence de l'espèce permet d'identifier la souche comme un champignon de l'espèce *Trichoderma atroviride.*

Les techniques moléculaires, en particulier le séquençage du gène *EF*1α, permettent d'identifier sans ambiguïté l'espèce comme un *Trichoderma atroviride* suite à une comparaison de la séquence du gène avec celle de l'espèce de référence. Par ailleurs, le test de détection appelé « test 11 » s'avère positif, confirmant ainsi l'appartenance de la souche à l'espèce *Trichoderma atroviride.*

En conclusion, cette souche de champignon se positionne ainsi dans la taxonomie des micro-organismes : *Eucaryota, Fungi, Ascomycota, Pezizomycotina, Sordariomycetes, Hypocreomycetidae, Hypocreales, Hypocreaceae, Hypocrea, Trichoderma atroviride.*

L'isolement du genre *Trichoderma* peut être réalisé à partir d'un échantillon de terre ou à partir de matières organiques. Le sol sera de préférence humide, cultivé ou maraîcher de manière à avoir une concentration importante de *Trichoderma.*

Après prélèvement, les échantillons sont séchés à température douce (20-25°C), broyés et homogénéisés puis conservés dans des sacs en plastique dans un endroit frais (4-5°C) en attendant de procéder à l'isolement. Bien qu'ils puissent être maintenus plusieurs mois, il est toutefois préférable de les traiter le plus rapidement possible.

L'isolement de la population de *Trichoderma,* en particulier *Trichoderma atroviride* AGR2, prélevée sera réalisé sur des milieux spécifiques choisis :
- le milieu de Davet constitué de :

| | |
|---|---|
| o Ca(NO₃)₂: | 1g, |
| o CaCl₂, 2H₂O : | 1 g, |
| o KNO₃: | 250 mg, |
| o MgSO₄, 7H₂O : | 250 mg, |
| o KH₂PO₄: | 125 mg, |
| o saccharose : | 2g, |
| o acide citrique : | 50 mg, |
| o gélose : | 25 g, |
| o eau distillée : | 1000 ml. |

Après autoclavage, le pH est ajusté à 4,5 avec du HCl 1 N avant d'ajouter au milieu tiède du sulfate de streptomycine (30 mg), du vinchlozoline (2,5 mg), et de l'alcool allylique (0,5 ml).
- le milieu TSM d'Elad et al comprenant :

| | |
|---|---|
| o MgSO₄, 7H₂O : | 200 mg, |
| o KH₂PO₄: | 900 mg, |
| o KCI : | 150 mg, |
| o NH₄NO₃: | 1 g, |
| o glucose: | 3g, |
| o chloramphénicol : | 250 mg, |
| o fénaminosulf : | 300 mg, |
| o quintozène : | 200 mg, |
| o rose bengale : | 150 mg, |
| o gélose : | 20 g, |
| o eau distillée : | 1000 ml. |

- le milieu TME de Papavizas constitué de :

| | |
|---|---|
| o glucose : | 1g, |
| o gélose : | 20 g, |
| o eau distillée : | 800 ml. |

Le mélange est ensuite mis à l'autoclave. Il est ajouté 200 ml de V-8, le pH doit être compris entre 3,8 et 4. Au milieu encore tiède, on ajoute du sulfate de néomycine (100 mg), de la bacitracine (100 mg), de la pénicilline G (100 mg), du chloronède (100 mg), de la nystatine (20 mg), de la chlortétracyline HCL (25 mg), et du propionate de sodium (500 mg).

Lorsque l'isolement est réalisé à partir d'un échantillon de terre, on utilise la technique de l'incorporation directe du sol. Le milieu d'isolement étant coulé et maintenu en surfusion en boîtes de Pétri à 37-40°C, une faible quantité de terre (5 à 15 mg) est saupoudrée et immédiatement dispersée dans le milieu, en agitant doucement jusqu'à solidification. Les cultures sont ensuite mises en incubation pendant quelques jours. Après repérage des colonies n'entrant pas en confluence, on procède à l'examen, au dénombrement puis à l'isolement des colonies.

Une pesée de l'échantillon de terre avant et après l'ensemencement permet de connaître précisément la masse de terre analysée et ainsi de calculer le nombre de propagules présentes par gramme de terre.
Pour l'isolement à partir de matières organiques, la technique des suspensions-dilutions est utilisée.
Ce principe consiste à mettre l'échantillon de sol en suspension dans de l'eau stérile, puis à incorporer les différentes dilutions de cette suspension dans le milieu d'isolement.
A cet effet, 10 grammes de terre sont ajoutés à 90 ml d'eau stérile avec une goutte de Tween. L'ensemble est mis sous agitation pendant 30 minutes pour constituer la solution-mère (dilution à 10⁻¹). Des prélèvements successifs de 10 ml de cette suspension mère puis des suivantes, ajoutés à 90 ml d'eau stérile vont constituer les dilutions suivantes (10⁻², 10⁻³... jusqu'à 10⁻⁶ en général).
Au moment de l'analyse, 10 ml de chaque solution sont versés dans un erlenmeyer contenant 90 ml de milieu sélectif d'isolement, maintenu en surfusion au bain-marie à 37-40°C. Après homogénéisation, les 100 ml sont répartis dans des boîtes de Pétri et placées en conditions propices à l'isolement de *Trichoderma.*
Après 3 à 5 jours d'incubation, on repère la dilution présentant un nombre de colonies suffisant mais sans confluence, ce qui aurait pour effet de compliquer l'isolement. La dilution de travail étant choisie, on peut procéder à l'examen, au dénombrement et à l'isolement des colonies.

Après repiquage sur milieu PDA, et exposition à la lumière pendant 4 à 5 jours, on procède à la purification, c'est-à-dire à l'isolement monosporal de chacune des colonies. Pour chaque colonie, les spores de couleur verte sont prélevées, mises en suspension dans l'eau stérile afin de les dissocier avant une mise en culture pour chaque spore.
Chaque colonie semblant la plus vigoureuse est choisie dans chaque boîte de Pétri puis repiquée sur une nouvelle boîte de Pétri et mise en collection.
Afin de sélectionner la souche susceptible de posséder le meilleur potentiel antagoniste, il a été choisi de réaliser un test de confrontation vis-à-vis d'une souche de *Botrytis cinerea,* qui est un pathogène polyphage de nombreuses cultures.

Le clone *Trichoderma atroviride AGR2* est retenu comme étant celui qui a la plus grande efficacité à l'égard de Botrytis. Ces résultats peuvent se résumer également à l'application de l'espèce *Trichoderma atroviride.*
Les boîtes de Pétri sont incubées à 20°C pendant quelques jours jusqu'à 2738, est retenu comme étant l'apparition d'une sporulation. Ces spores sont prélevées au moyen d'une oëse, puis conservées en mycothèque.

Une étude a été réalisée sur la souche *Trichoderma atroviride AGR2,* qui a été repiquée tous les 4 jours jusqu'à la vingtième génération. La méthode de RAPD (Random Amplified Polymorphic DNA) n'a pas permis de mettre en évidence une quelconque mutation de la souche.
En conclusion, la souche *Trichoderma atroviride AGR2* présente une stabilité génétique, permettant son utilisation dans les vignes et autres cultures.

Plusieurs études ont été menées afin d'évaluer l'efficacité du champignon contre différents agents pathogènes.
Le pouvoir antagonisme de *Trichoderma atroviride,* et en particulier de la souche *AGR2,* est mesuré à l'égard des champignons associés aux maladies du bois de la vigne : *Phaeomoniella chlamydospora, Phaeoacremonium aleophilum, Fomitiporia punctata, Eutypa lata, Botryosphaeria obtusa, Botryosphaeria parva, Botryosphaeria stevensii.*

Ces différents champignons, isolés à partir de ceps atteints d'esca, d'eutypiose, de dépérissement de la Syrah, de BDA ou de greffés-soudés, sont conservés à 4°C en tube sur milieu malt-agar (15 g de malt et 20 g de gélose pour un litre d'eau. Le milieu est stérilisé à 120°C pendant 20 mn). Ils sont repiqués à partir des tubes de conservation sur milieu malt-agar dans des boîtes de Pétri pour la multiplication et les tests de confrontation avec *Trichoderma atroviride* AGR2. Par ailleurs, une souche de référence de *Trichoderma harzianum* a été incluse dans ce test.

Lors de la mise en confrontation, deux implants mycéliens de 6 mm de diamètre des champignons à étudier sont prélevés d'une colonie à croissance active sur milieu malt et disposés dans la boîte de Pétri de façon diamétralement opposée. La distance entre les deux implants est de 45 mm.
Du fait de la croissance lente de certains champignons, l'ensemencement de ceux-ci est avancé de 8 jours (cas de *E. lata, F. punctata, P. aleophilum*) ou de 15 jours (cas du *P. chlamydospora*) par rapport au *Trichoderma* mis en confrontation. Les boîtes sont mises à incuber à 25°C. Pour chaque couple, trois répétitions sont effectuées. Le témoin correspond à la croissance du champignon seul (sans la présence du *Trichoderma).*
Pendant la première semaine, les boîtes sont observées et notées tous les jours. Ces opérations sont ensuite réalisées toutes les semaines. Les notations consistent à évaluer l'inhibition de la croissance de la colonie mycélienne.

Le taux d'inhibition, exprimé en mm, correspond à la différence entre le rayon R1 de la colonie de la boîte témoin et le rayon R2 qui est situé sur la droite reliant le centre des deux protagonistes en confrontation (I = R1 - R2). Le taux d'inhibition est calculé au bout de 4 jours pour *B. parva,* 5 jours pour les B. *obtusa* et *B. stevensii,* 7 jours pour *E. lata* et *F. punctata,* 9 jours pour P. *chlamydospora* et 12 jours pour *P. aleophilum.*

La lecture des résultats se fait également par observation des mycéliums en confrontation : présence d'une zone d'inhibition, recouvrement d'un mycélium par un autre, compétition spatiale, zones de mélanisation, fructifications...

A la fin des confrontations, chaque implant est remis sur un milieu de culture malt (15 g de malt et 20 g de gélose pour un litre d'eau. Le milieu est stérilisé à 120°C pendant 20 mn). La reprise ou non de la colonie est alors notée.

Les résultats sont représentés dans le tableau II

**Tableau II - Taux d'inhibition (exprimé en mm) de la croissance mycélienne des différents champignons impliqués dans les maladies du bois de la vigne face à la souche de Trichoderma atroviride AGR2 (mesuré au bout de 4 à 12 jours de confrontation)**

| MICRORGANISMES Pathogènes | Rayon de la colonie témoin | Taux d'inhibition (mm) | |
|---|---|---|---|
| | | *T. atroviride AGR2* | *T*. *harzianum* (ref.) |
| *Phaeomoniella chlamydospora LR47* | 14 | 3 | 1,7 |
| *Phaeomoniella chlamydospora LR81* | 16 | 5 | 3 |
| *Phaeoacremonium aleophilum LR3* | 17,5 | 7,7 | 1,7 |
| *Phaeoacremonium aleophilum LR23* | 15,2 | 3,5 | -6,8 |
| *Fomitiporia punctata LR14* | 22,7 | 8,3 | 3,7 |
| *Fomitiporia punctata LR30* | 26,7 | 10 | 5 |
| *Eutypa lata cc260* | 36 | 7,7 | 1,5 |
| *Eutypa lata MT247* | 38,7 | 10,7 | 9,4 |
| *Botryosphaeria obtusa F98-1* | 48,2 | 25,2 | 22,6 |
| *Botryosphaeria obtusa F99-8* | 43,9 | 23,9 | 19,9 |
| *Botryosphaeria parva Bd021* | 48,6 | 21,9 | 18,3 |
| *Botryosphaeria stevensii Bs001* | 54,1 | 29,4 | 25,7 |

L'antagonisme de *Trichoderma atroviride* AGR2, et en général celui de l'espèce *Trichoderma atroviride,* est donc réel à l'égard des champignons pathogènes *Phaeomoniella chlamydospora, Phaeoacremonium aleophilum, Fomitiporia punctata, Eutypa lata, Botryosphaeria obtusa, Botryosphaeria parva, Botryosphaeria stevensii.* La souche *Trichoderma atroviride* AGR2 inhibe la croissance des isolats et recouvre très souvent les colonies en produisant des fructifications à leurs surfaces.
La remise en culture des implants de *Phaeomoniella chlamydospora, Phaeoacremonium aleophilum, Fomitiporia punctata, Eutypa lata, Botryosphaeria obtusa, Botryosphaeria parva, Botryosphaeria stevensii* montre que ceux-ci se redéveloppent rarement.

D'autres tests ont permis également de montrer le rôle antagoniste de la souche selon l'invention envers d'autres champignons pathogènes de la vigne tels que *Stereum hirsutum, Phomopsis viticola, Phellinus ignarius, Phellinus viticola, Armillaria mella.*

En revanche, les taux d'inhibition de la souche de référence de *Trichoderma harzianum* sont inférieurs à celui de la souche *Trichoderma atroviride* AGR2, montrant ainsi un antagonisme supérieur que celui du genre *Trichoderma harzanium.*

L'observation du comportement de la référence *Trichoderma harzianum* montre qu'elle ne recouvre pas la colonie de *Phaeomoniella chlamydospora* ni celle de *Phaeoacremonium aleophilum.* Les deux protagonistes restent seulement en contact. La remise en culture de l'implant de la colonie testée montre que les isolats pathogènes ne se développent pas.
Par contre, elle recouvre la colonie de *Fomitiporia* et produit des fructifications à sa surface. La remise en culture de l'implant de la colonie testée montre que les isolats pathogènes Fp 14 et Fp 30 ne se développent pas
Le taux d'inhibition de *Trichoderma harzianum* à l'encontre de la souche *d'Eutypa lata* cc260 est très faible : la souche ne recouvre pas la colonie pathogène, qui par la suite se développe sur la colonie de *Trichoderma.* Par contre le taux d'inhibition vis à vis de la souche *d'Eutypa lata* MT247 peut être considérée comme similaire à *Trichoderma atroviride.*
La souche de *Trichoderma harzianum* ne colonise pas la culture des *Botryosphaeria.* Par la suite, *B. obtusa, B. parva* et *B. stevensii* recouvrent *Trichoderma harzianum.* La remise de l'implant de la colonie testée sur milieu de culture montre que *B. obtusa, B. parva* et *B. stevensii* se développent.

Ainsi donc, si *Trichoderma harzianum* exerce un certain taux d'inhibition, celui-ci est le plus souvent assez faible à l'égard des différents champignons du bois et ne semble pas un bon antagoniste pour la lutte contre les maladies du bois, contrairement à *Trichoderma atroviride.*

Enfin, le pouvoir antagonisme de l'espèce *Trichoderma* atroviride, et en particulier celui de la souche *Trichoderma atroviride* AGR2 a été également évalué à l'égard de champignons pathogènes des autres cultures: *Sclérotinia sclerotirum, Sclérotinia minor, Botrytis cinerea, Phomopsis, Microdocchium nivale, Fusarium culmorum*

Ces différents champignons, isolés à partir de cultures atteintes de sclérotinia (Colza, salades), botrytis (vigne), fusariose (céréales), Phomopsis (tournesol), sont conservés à 4°C en tube sur milieu malt-agar (15 g de malt et 20 g de gélose pour un litre d'eau. Le milieu est stérilisé à 120°C pendant 20 mn). Ils sont repiqués à partir des tubes de conservation sur milieu malt-agar dans des boîtes de Pétri pour la multiplication et les tests de confrontation avec *Trichoderma atroviride*.

Lors de la mise en confrontation, deux implants mycéliens de 6 mm de diamètre des champignons à étudier sont prélevés d'une colonie à croissance active sur milieu malt et disposés dans la boîte de Pétri de façon diamétralement opposée. La distance entre les deux implants est de 50 mm. Les boîtes sont mises à incuber à 25°C. Pour chaque couple, trois répétitions sont effectuées. Le témoin correspond à la croissance du champignon seul (sans la présence du *Trichoderma).*
Pendant 6 jours, les boîtes sont observées et notées. Les notations consistent à évaluer l'inhibition de la croissance de la colonie mycélienne. Le taux d'inhibition, exprimé en mm, correspond à la différence entre le rayon R1 de la colonie de la boîte témoin et le rayon R2 qui est situé sur la droite reliant le centre des deux protagonistes en confrontation (I = R1 - R2). Le taux d'inhibition est calculé au bout de 6 jours.
La lecture des résultats se fait également par observation des mycéliums en confrontation : présence d'une zone d'inhibition, recouvrement d'un mycélium par un autre, compétition spatiale, zones de mélanisation, fructifications...

Les résultats sont représentés dans le tableau III

**Tableau III - Taux d'inhibition (exprimé en mm) de la croissance mycélienne des différents champignons pathogènes des cultures face à la souche de Trichoderma atroviride AGR2 (mesuré au bout de 4 à 6 jours de confrontation)**

| MICRORGANISMES PATHOGENES | Rayon de la colonie témoin | Taux d'inhibition (mm) | |
|---|---|---|---|
| | | T. atroviride AGR2 | T. harzianum (ref.) |
| *Sclérotinia sclerotirum* | 83 | 15.0 | 11.9 |
| *Sclérotinia minor* | 85 | 36.7 | 22.1 |
| *Botrytis cinerea* | 77,3 | 29.6 | 27.8 |
| *Phomopsis hélianthi* | 56,5 | 12.8 | 6.7 |
| *Microdocchium nivale* | 32 | 5.0 | 3.2 |
| *Fusarium culmorum* | 45,5 | 9.3 | 10.2 |

L'antagonisme de Trichoderma est donc réel à l'égard des champignons pathogènes *Sclérotinia sclerotirum, Sclérotinia minor, Botrytis cinerea, Phomopsis viticola, Microdocchium nivale, Fusarium culmorum.* La souche *Trichoderma atroviride* AGR2 inhibe la croissance des isolats et recouvre très souvent les colonies en produisant des fructifications à leurs surfaces. La remise en culture des implants de *Sclérotinia sclerotirum, Sclérotinia minor, Botrytis cinerea, Phomopsis viticola, Microdocchium nivale, Fusarium culmorum* montre que ceux-ci ne se redéveloppent pas.

Enfin, lorsque l'espèce *Trichoderma atroviride,* et en particulier la souche de *Trichoderma atroviride AGR2,* sont incorporées dans une terre préalablement stérilisée, c'est-à-dire en l'absence de pathogènes, il a été observé une action de stimulation de la germination et de la croissance des salades.
L'essai a été réalisé dans des barquettes de 672 cm2, remplies de terre stérilisée et ensemencées avec *Trichoderma atroviride AGR2* à 3 doses (33,6, 67,2 et 672 mg de produit technique par barquette) avant de semer la salade (variété laitue Titan) à raison de 20 salades par barquette. Un arrosage régulier est assuré chaque jour pendant 35 jours.
Les résultats sont représentés dans le tableau IV

**Tableau IV - Stimulation de la germination et de la croissance de la salade par incorporation de la souche de Trichoderma atroviride AGR2 dans une terre stérilisée (indice 100 pour le témoin).**

| **Critère d'observation** | **Témoin** | **Dose A** (33,2 mg) | **Dose B** (67,2 mg) | **Dose C** (672 mg) |
|---|---|---|---|---|
| Germination à J+7 | 100 | 146,2 | 138,5 | 115,4 |
| Nombre de feuilles à J+33 | 100 | 115,1 | 125,8 | 112,1 |
| Longueur des feuilles à J+33 | 100 | 137,1 | 146,7 | 123,8 |

L'effet de stimulation agit dès la première dose (33,2 mg/barquette). La concentration 20 fois supérieure (672 mg) montre une bonne sélectivité puisque la stimulation est toujours présente; cependant, celle-ci est moins importante que pour les doses A et B.

Des essais de résistance et de sensibilité de la souche *Trichoderma atroviride AGR2* face à des agents anti-microbiens, utilisés comme fongicides des cultures ont été réalisés. Outre son caractère pratique pour une utilisation du clone I-2738 par les agriculteurs, les résultats peuvent également permettre une meilleure connaissance de *Trichoderma atroviride AGR2.*
Les tests de croissance in vitro du *Trichoderma atroviride AGR2* sont réalisés sur gélose enrichie de produits commerciaux contenant les substances fongicides étudiées.
La méthodologie employée est la suivante :
- constitution d'une solution de 100 ml pour chaque produit à tester dans laquelle est diluée le produit commercial
- étalement de 0,126 ml de la solution sur un milieu de culture Sabouraud (peptone mycologique à 10 g.L⁻¹, glucose à 40 g.L⁻¹, agar à 15 g.L⁻¹)
- dépôt d'un implant mycélien de *Trichoderma atroviride AGR2* de 3 mm et mise en culture à température ambiante.
- mesure de la croissance en pourcentage du témoin

Les résultats sont représentés dans le tableau V

**Tableau V - Inhibition de la croissance mycélienne la souche de Trichoderma atroviride AGR2 par différents produits phytosanitaires (croissance exprimée en pourcentage du témoin)**

| Produit commercial (marques déposées) | Matière(s) active(s) | Concentration de la solution (/100ml) | J+1 | J+2 | J+3 |
|---|---|---|---|---|---|
| Cursor^{®} | Cymoxanil+Folpel +Mancozèbe +Cuivre | 1,25 g | 105,46 | 103,38 | 106,85 |
| Microthiol^{®} | Soufre | 6,25 g | 90,00 | 85,63 | 100,00 |
| Round up^{®} | Glyphosate | 6 ml | 95,95 | 94,16 | 100,00 |
| Stroby DF^{®} | Krésoxy-méthyl | 0,1 g | 74,58 | 81,88 | 100,00 |
| Témoin^{®} | - | - | 100,00 | 100,00 | 100,00 |
| Acrobat^{®} | Diméthomorphe +Mancozèbe | 1,25 g | 62,65 | 68,54 | 88,63 |
| Légend^{®} | Quinoxyfen | 0,1 ml | 106,68 | 90,15 | 88,05 |
| Scala^{®} | Pyriméthanil | 1,25 ml | 95,14 | 90,51 | 87,25 |
| Valiant^{®} | Fosétyl-Al+Folpel +Cymoxanil | 1,5 g | 66,22 | 76,61 | 86,76 |
| Hoggar^{®} | Spiroxamine | 0,3 ml | 52,00 | 61,02 | 81,57 |
| Odéna^{®} | Folpel +Iprovanicarbe | 1 g | 66,16 | 65,17 | 81,17 |
| Tairel F^{®} | Bénalaxyl+Folpel | 1,25 ml | 58,66 | 60,41 | 76,86 |
| Surfassol^{®} | Dichlobénil | 50 g | 38,07 | 50,67 | 76,08 |
| Dobran^{®} | Krésoxy-méthyl | 0,1 g | 77,18 | 73,02 | 74,93 |
| Olymp^{®} | Fluzilazole | 0,15 ml | 59,13 | 62,54 | 72,73 |
| Basta^{®} | Glufosinate-ammonium | 1,25 ml | 77,18 | 64,72 | 64,25 |
| Panthéos^{®} | Diméthomorphe +Folpel | 1 g | 79,96 | 61,05 | 58,38 |
| Karathane^{®} | Dinocap | 0,3 ml | 89,19 | 64,51 | 55,13 |
| Score^{®} | Difénoconazole | 0,06 ml | 66,52 | 48,07 | 48,45 |
| Sumisclex^{®} | Procymidone | 0,15 ml | 49,65 | 29,35 | 24,42 |
| Sekoya^{®} | Fluazinam | 0,75 ml | 33,99 | 20,15 | 17,93 |
| Sting^{®} | Glyphosate | 5,4 ml | 43,06 | 15,49 | 12,19 |
| Jonk^{®} | Diéthofencarbe +Carbendazime | 1 ml | 0,00 | 0,00 | 0,00 |
| Geoxe^{®} | Fludioxonil | 0,5 g | 0,00 | 0,00 | 0,00 |

Dans le but d'obtenir un produit technique servant de base à la production d'un produit phytosanitaire destiné à protéger les cultures, une technique de production a été mise au point. Elle se déroule en deux étape : la première concerne la réalisation de l'inoculum primaire, qui a lieu en laboratoire. La seconde phase consiste en la réalisation propre du produit technique.

Dans un premier temps, la production de l'inoculum primaire de *Trichoderma atroviride AGR2* en laboratoire est réalisée sur boîtes de Pétri, sur un milieu de culture Sabouraud (peptone mycologique à 10 g.L⁻¹, glucose à 40 g.L⁻¹, agar à 15 g.L⁻¹) ou un milieu malt-agar. On ajoute à ce milieu du chloramphénicol à une concentration de 2 mL.L⁻¹ d'une solution à 5 g/50 mL d'éthanol, le chloramphénicol empêchant le développement de bactéries indésirables. La souche utilisée pour la production de l'inoculum primaire est la souche mère déposée à l'Institut Pasteur.
Après culture et observation visuelle des champignons présents dans la boîte de Pétri, les boîtes de Pétri éventuellement polluées par des micro-organismes autres que la souche *Trichoderma atroviride AGR2* sont éliminées.
La seconde phase consiste à préparer le produit technique proprement dit. Pour cela, les spores de *Trichoderma atroviride AGR2,* contenues dans les boîtes de Pétri sont inoculées sur un substrat organique tel que des pulpes de betterave, de pomme de terre, des flocons d'avoine, d'orge ou de maïs, de malt-agar ou PDA stériles. Afin de favoriser la sporulation de la souche *Trichoderma atroviride AGR2,* du chlorure de calcium à raison de 4 g.kg⁻¹ de support organique est ajouté.
La réalisation de cette phase se fait de la manière suivante : le contenu en spores des boîtes de Pétri est mélangé dans un système de brassage avec de l'eau et du CaCl₂. Le mélange est réparti sur des plateaux pour incubation en enceinte climatisée à 20-25°C, où il est entretenu une hygrométrie et une aération nécessaires à la croissance du *Trichoderma atroviride.*
Après 4 à 6 jours d'incubation, l'ensemble est séché et micronisé sous forme de poudre pour constituer le produit technique, riche en spores de *Trichoderma atroviride AGR2.* Un comptage en cellule de Malassez détermine sa concentration en spores : selon les supports organiques utilisés, celle-ci va de 10⁸ à 10¹⁰ spores par gramme.
Un procédé avantageux revendiqué par la présente invention consiste à utiliser un substrat organique extrudé, permettant une croissance rapide de *Trichoderma* et des concentrations élevées en spores par gramme.
Cette poudre constitue la base de produits phytosanitaires multiples adaptés aux différents usages souhaités.

Différentes études ont été menées afin de vérifier l'inocuité de la souche *Trichoderma atroviride AGR2* sur l'homme et son environnement, que ce soit lors de la fabrication du produit technique ou lors de son application sur les cultures.
Ainsi, quatre études réalisées sur des vers de terre, poissons, daphnies et algues ont montré la parfaite sûreté de la souche pour l'environnement.
Aucun effet de la souche *Trichoderma atroviride AGR2* sur les mycorhizes, les champignons nématophages, les nématodes n'a été rapporté.

La croissance mycélienne de la souche *Trichoderma atroviride AGR2* est optimale à une température comprise entre 20 et 25°C. Par contre, sa croissance est nulle à 37°C, laissant supposer qu'elle ne peut pas se développer *in vivo* chez l'homme.

Cependant, des tests complémentaires suivants ont été réalisés afin d'évaluer les effets que peut avoir le produit technique sur les mammifères et par extrapolation l'homme. Ces tests concernent :
- la sensibilisation cutanée,
- la sensibilisation par inhalation,
- la toxicité aiguë par voie orale,
- la toxicité aiguë par voie intrapéritonéale,
- la toxicité aiguë par voie dermique,
- l'irritation aiguë sur les yeux,
- l'aberration chromosomique des lymphocytes humains,
- la toxicité, pathogénicité et infectiosité aiguës par voie intratrachéale.
Chacun de ces tests a révélé que la souche selon l'invention est parfaitement sûre et qu'elle ne produit aucun métabolite sensible pouvant représenter un risque perceptible pour la santé humaine ou pour l'environnement.
Par conséquent, *Trichoderma atroviride AGR2* et le produit technique ne présentent aucun effet toxique, pathogène et infectieux en cas d'exposition.

Un usage avantageux de la présente invention, c'est-à-dire la souche *Trichoderma atroviride AGR2,* est son utilisation dans la formulation d'un produit phytosanitaire comme matière active. Ainsi, la poudre obtenue par le mode de production décrit précédemment peut être mélangée ou non à un ou plusieurs composants tels que : une ou plusieurs autres matières actives, un ou plusieurs substrats nutritifs et/ou organiques et/ou minéraux, un ou plusieurs mouillants, un ou plusieurs dispersants, un ou plusieurs anti-mousse, etc... de manière à obtenir un produit final aux caractéristiques et à la concentration en spores souhaitées.

Le produit de traitement phytosanitaire à base de la souche naturelle *Trichoderma atroviride AGR2* est destiné au traitement des maladies fongiques touchant la vigne telles que l'eutypiose, l'esca, le black dead arm, l'excoriose, l'armillaire et botrytis, dans la mesure où la souche agit sur ces champignons pathogènes attaquant la vigne.

Ainsi, selon un premier usage, le produit phytosanitaire sera appliqué par pulvérisation ou badigeonnage sur les ceps de vigne, après la taille ou à toute autre période de l'année pour effectuer une protection contre les maladies de bois.

Ainsi, une étude au vignoble, confirmant les résultats précédents réalisés in vitro a été faite.
En hiver, le jour de la taille, le produit phytosanitaire à base de *Trichoderma atroviride AGR2* ou de *Trichoderma atroviride* est pulvérisé sur les plaies à des concentrations variables de 5%, 2,5% et 1%. Deux lots de sarments sont prélevés, d'abord 7 jours après la pulvérisation, puis à 45 jours. A partir du front de taille, le bois est découpé en 8 lamelles de 1 millimètre d'épaisseur. Ces lamelles sont déposées sur des boîtes de Pétri contenant un milieu gélosé afin d'être artificiellement contaminées avec les spores des deux champignons pionniers pathogènes : *Eutypa lata* et *Phaeomoniella chalmydospora.*
Lorsque le produit phytosanitaire est appliqué à une dilution à 2,5%, il présente une bonne capacité de contrôle du développement du champignon pathogène *Eutypa lata.* Le produit phytosanitaire reste efficace sur une période de 45 jours minimum après la taille.
Pour le contrôle de *Phaeomoniella chlamydospora,* une dilution à 2,5% du produit phytosanitaire est suffisante, qui reste efficace même 45 jours après la taille.

Cette étude démontre également que l'efficacité de la pulvérisation du produit phytosanitaire à base de *Trichoderma atroviride AGR2* est supérieure à celle d'une solution à 1% d'un produit chimique contenant 125 g/l de Flusilazole + 250 g/l de Carbendazime, dérivé de celui habituellement utilisé pour le traitement de l'esca.

L'espèce *Trichoderma atroviride* et la souche *Trichoderma atroviride AGR2,* sous forme d'un produit phytosanitaire, agissent bien sur les champignons pathogènes *Phaemoliella chlamydospora* et *Eutypa lata*.
Dans d'autres essais, la pulvérisation d'une composition à base de *Trichoderma atroviride AGR2* sur des plaies de taille montre qu'il colonise le bois jusqu'à une profondeur de 15 millimètres, et reste présent pendant une période de 6 à 10 mois.

Selon un second usage, le produit selon l'invention pourra être appliqué au sol et/ou être utilisé pour praliner (enrober) les racines des jeunes plants avant la plantation afin de stimuler la croissance racinaire et lutter contre l'Armillaire.

Selon un troisième usage, le produit selon l'invention peut être pulvérisé sur les grappes en végétation pour lutter contre le Botrytis, agent responsable de la pourriture des grappes. En effet, dans ce cas, la souche *Trichoderma atroviride AGR2* doit lutter contre les effets nocifs des rayons UV. Cependant, elle possède un gène phr1 induit par la lumière bleue, s'exprimant à la fois dans le mycélium végétatif car il manque des pigments photoprotecteurs, et dans les conidiophores. Ce gène code pour des photolyases, catalysant la réparation de l'ADN.
Par ailleurs, la lumière visible induit le développement de spores pigmentées et résistantes. Il est ainsi possible d'envisager une utilisation de la souche *Trichoderma atroviride AGR2* lors d'un traitement aérien des cultures, sans risquer un phénomène de photodégradation excessif par les rayons UV.

La souche *Trichoderma atroviride AGR2* colonise également le sol, en particulier lorsqu'il est maraîcher, et surtout lorsqu'un apport en matière organique a été réalisé. C'est en effet un champignon connu pour présenter une bonne aptitude au développement saprophytique dans le sol.

Selon un quatrième usage, les produits selon l'invention pourra donc être appliqué au sol et/ou être utilisé pour praliner (enrober) les racines des jeunes plants avant la plantation afin de lutter contre les maladies d'infection des racines, notamment pour les productions maraîchères, tels que Sclérotinia, Rhizoctone, Botrytis, Fonte de semis due aux fusarium, Pithium, etc...
Comme il a été observé, lors d'essais en terrine, une croissance plus rapide des plants de salades, il pourra avantageusement être utilisé pour stimuler la croissance des plants et des racines, et lutter contre le syndrome de la fatigue des sols, notamment sur fraisiers

II s'avère que la souche *Trichoderma atroviride AGR2* ne présente aucun risque pour l'environnement, dans la mesure où la souche ne présente aucune toxicité, ni pouvoir infectieux sur aucun des organismes vivants avec lequel le produit phytosanitaire pourrait entrer en contact lors de son utilisation, et notamment aucune incidence sur l'homme.
Le produit phytosanitaire peut donc être utilisé comme moyen alternatif de protection des cultures, dans le cadre de traitements biologiques, seul ou en compléments des traitements chimiques en privilégiant dans ces derniers les plus respectueux de la souche *Trichoderma atroviride* AGR2.

Le fait d'utiliser le produit technique ou phytosanitaire sous forme d'une poudre mouillable appliquée par pulvérisation facilite le traitement des vignes, dans la mesure où il est plus rapide d'utilisation que le badigeon chimique employé habituellement pour le traitement des plaies de taille et qui représente opération minutieuse, longue et fastidieuse.

Bien que l'invention ait été décrite avec des moyens de réalisation particuliers, elle comprend tous les équivalents techniques des moyens décrits.

## Revendications

1. Souche de Trichoderma atroviride **caractérisée en ce qu'**il s'agit de la souche Trichoderma atroviride AGR2 déposée à la Collection Nationale de Cultures de Micro-organismes (CNCM) de l'Institut Pasteur sous le numéro I-2738.

2. Procédé d'isolement de la souche Trichoderma atroviride AGR2 selon la revendication 1, **caractérisé en ce qu'**il comprend les étapes suivantes :
a. prélèvement de la souche Trichoderma atroviride AGR2 sur un échantillon de terre,
b. saupoudrage et dispersion de l'échantillon prélevé à l'étape ci-dessus sur une boîte de Pétri à 37-40°C contenant un milieu d'isolement,
c. agitation douce jusqu'à solidification du milieu,
d. incubation pendant plusieurs jours,
e. examen, dénombrement et isolement des colonies de Trichoderma atroviride AGR2.

3. Procédé d'isolement de la souche Trichoderma atroviride AGR2 selon la revendication 1, **caractérisé en ce qu'**il comprend les étapes suivantes :
a. prélèvement d'un échantillon de terre composé de matières organiques,
b. mise en suspension dudit échantillon (10g) dans 90 mi d'eau stérile avec une goutte de Tween,
c. agitation pendant 30 minutes,
d. prélèvement de 10 ml de cette solution puis dilutions successives jusqu'à 10⁻⁶,
e. versement de 10 ml de chaque solution dans 90 ml de milieu d'isolement, maintenu en surfusion au bain-marie à 37-40°C,
f. homogénéisation et répartition dans des boîtes de Pétri,
g. incubation pendant 3 à 5 jours,
h. examen, dénombrement et isolement des colonies de Trichoderma atroviride AGR2.

4. Procédé d'isolement selon l'une des revendications 2 ou 3, **caractérisé en ce qu'**il comprend les étapes suivantes :
a. repiquage des colonies isolées sur milieu PDA, et exposition à la lumière pendant 4 à 5 jours,
b. prélèvement des spores de couleur verte,
c. mise en suspension des spores dans l'eau stérile,
d. choix de la colonie la plus vigoureuse,
e. repiquage dans une boîte de Pétri,
f. test de confrontation avec une souche de Botrytis cinerea.

5. Procédé d'isolement selon l'une des revendications 2 ou 3, **caractérisé en ce que** le milieu d'isolement est le milieu de Davet, constitué de :
- Ca(NO₃)₂: 1g,
CaCl₂, 2H₂O: 1g,
- KNO₃: 250 mg,
- MgSO₄, 7H₂O: 250 mg,
- KH₂PO₄: 125 mg,
- saccharose : 2g,
- acide citrique : 50 mg,
- gélose : 25 g,
- eau distillée : 1000 ml ;
le milieu étant ensuite mis à l'autoclavage, avec ajustement du pH à 4,5 avec du HCl 1 N ; puis ajout au milieu tiède de sulfate de streptomycine (30 mg), de vinchlozoline (2,5 mg), et d'alcool allylique (0,5 ml).

6. Procédé d'isolement selon l'une des revendications 2 ou 3, **caractérisé en ce que** le milieu d'isolement est le milieu TSM d'Elad et al comprenant :
- Mg SO₄, 7H₂O: 200 mg,
- KH₂PO₄: 900 mg,
- KCl : 150 mg,
- NH₄NO₃: 1g,
- glucose: 3g,
- chloramphénicol : 250 mg,
- fénaminosulf : 300 mg,
- quintozène : 200 mg,
- rose bengale : 150 mg,
- gélose : 20 g,
- eau distillée 1000 ml.
le milieu est ensuite mis à l'autoclave, et il y est ajouté 200 ml de V-8, le pH devant être compris entre 3,8 et 4 ; quand il est encore tiède, le milieu se voit ajouter du sulfate de néomycine (100 mg), de la bacitracine (100 mg), de la pénicilline G (100 mg), du chloronède (100 mg), de la nystatine (20 mg), de la chlortétracyline HCL (25 mg), et du propionate de sodium (500 mg).

7. Procédé d'isolement selon l'une des revendications 2 ou 3, **caractérisé en ce que** le milieu d'isolement est le milieu d'isolement est le milieu TME de Papavizas constitué de :
- glucose : 1 g,
- gélose : 20 g,
- eau distillée : 800 ml,
qui est ensuite mis à l'autoclave, puis on y ajoute 200 ml de V-8, le pH devant être compris entre 3,8 et 4 ; quand il est encore tiède, on y ajoute du sulfate de néomycine (100 mg), de la bacitracine (100 mg), de la pénicilline G (100 mg), du chloronède (100 mg), de la nystatine (20 mg), de la chlortétracyline HCL (25 mg), et du propionate de sodium (500 mg).

8. Procédé d'obtention d'un produit à base de Trichoderma atroviride AGR2 obtenue selon le procédé de l'une des revendications 2 à 7, **caractérisé en ce qu'**il consiste en différentes étapes :
a. production de la souche Trichoderma atroviride AGR2 réalisée sur boîtes de Pétri contenant un milieu de culture Sabouraud ou malt-agar, contenant du chloramphénicol,
b. isolement des souches de Trichoderma atroviride AGR2, se présentant sous la forme de spores,
c. inoculation de la souche du champignon sur des pulpes de betterave, de pomme de terre, des flocons d'avoine, d'orge ou de maïs, de malt-agar ou PDA stériles,
d. extrusion de l'ensemble du substrat, permettant d'obtenir une concentration élevée de spores,
e. ajout de chlorure de calcium.
f. incubation en chambre non stérile pendant 4 à 6 jours,
g. séchage et micronisation sous forme de poudre.

9. Utilisation de la souche Trichoderma atroviride AGR2 selon la revendication 1 ou obtenue selon le procédé d'une des revendications 2 à 8 dans la lutte contre les maladies des plantes.

10. Utilisation de la souche Trichoderma atroviride AGR2 selon la revendication 1 ou obtenue selon le procédé d'une des revendications 2 à 8, en tant que produit phytosanitaire par pulvérisation, ou badigeonnage, ou par pralinage des racines.

11. Utilisation de la souche Trichoderma atroviride AGR2 selon la revendication 1 ou obtenue selon le procédé d'une des revendications 2 à 8, en tant que produit phytosanitaire constitué de ce seul micro-organisme.

12. Utilisation de la souche Trichoderma atroviride AGR2 selon la
revendication 1 ou obtenue selon le procédé d'une des revendications 2 à 8, en tant que produit phytosanitaire en association avec d'autres fongicides chimiques.

13. Utilisation de la souche Trichoderma atroviride AGR2 selon la revendication 1 ou obtenue selon le procédé d'une des revendications 2 à 8, en tant que moyen de lutte contre l'esca, l'eutypiose, le black dead arm, l'excoriose et le sclérotinia.

14. Utilisation de la souche Trichoderma atroviride AGR2 selon la revendication 1 ou obtenue selon le procédé d'une des revendications 2 à 8, en tant que produit phytosanitaire agissant contre les champignons pathogènes tels que Eutypa lata, Phaeomoniella chlamydospora, Phaeomoniella aleophilum, Fomitiporia punctata, Stereum hirsutum, Botryosphaeria dothidea et stevensii, Phomopsis viticola, Botrytis cinerea, Sclerotinia minor, sclerotiorum et cepivorum, Microdocchium nivale, Armillaria mella, Phomopsis hélianthi.

15. Utilisation de la souche Trichoderma atroviride AGR2 selon la revendication 1 ou obtenue selon le procédé d'une des revendications 2 à 8, en tant que produit destiné à la stimulation racinaire, de la germination et de la croissance des plantes, en particulier des salades.

16. Utilisation de la souche Trichoderma atroviride AGR2 selon la revendication 1 ou obtenue selon le procédé d'une des revendications 2 à 8, en tant qu'élément colonisant le bois sur les premiers centimètres.

## Patentansprüche

1. Stamm von *Trichoderma atroviride* **dadurch gekennzeichnet, dass** der Stamm in der Nationalen Sammlung von Mikroorganismenkulturen (Collection Nationale de Cultures de Micro-organismes CNCM) des Pasteur Instituts unter dem Nummer I- 2738 *Trichoderma atroviride AGR2* eingeschrieben ist.

2. Verfahren zur Isolierung des Stammes *Trichoderma atroviride AGR2* laut dem "1. Anspruch", **dadurch gekennzeichnet, dass** es die folgenden Schritte umfasst :
a. Entnahme von *Trichoderma atroviride* aus einer Bodenprobe,
b. Streuung und Dispersion der Bodenprobe in einer Petrischale bei 37-40 ° C, die ein Isolierungsmedium enthält,
c. vorsichtiges Rühren bis zur Verfestigung des Mediums,
d. Inkubation während mehreren Tagen,
e. Prüfung, Aufzählung und Isolierung der Kolonien von *Trichoderma atroviride AGR2.*

3. Verfahren zur Isolierung des Stammes *Trichoderma atroviride AGR2* laut dem "Anspruch 1", **dadurch gekennzeichnet, dass** es die folgenden Schritte umfasst :
a. Entnahme einer aus organischen Materialien zusammengesetzten Bodenprobe,
b. Aufwirbelung der Probe (10 g) in 90 ml sterilem Wasser mit einem Tropfen Tween,
c. Rühren während 30 Minuten,
d. Entnahme von 10 ml dieser Lösung und aufeinanderfolgende Verdünnungen bis einer Konzentration von 10⁻⁶,
e. Gießen von 10 ml jeder Lösung in 90 ml des Isolierungsmediums, dass im Wasserbad bei 37-40 ° C gehalten wird,
f. Homogenisierung und Verteilung in Petrischalen,
g. Inkubation für einen Zeitraum von 3 bis 5 Tagen,
h. Prüfung, Aufzählung und Isolierung der Kolonien von *Trichoderma atroviride AGR2.*

4. Verfahren zur Auswahl des *Trichoderma atroviride* Stammes, der laut einem der Ansprüche 2 oder 3 erhalten wurde, **dadurch gekennzeichnet, dass** es die folgenden Schritte umfasst :
a. Transplantation der isolierten Kolonien auf das PDA Medium und Belichtung für einen Zeitraum von 4 bis 5 Tagen
b. Entnahme der grünen Sporen,
c. Aufwirbelung der Sporen in sterilem Wasser,
d. Auswahl der stärksten Kolonie,
e. Umpflanzen in einer Petrischale,
f. Vergleichstest mit einem Stamm von *Botrytis cinerea.*

5. Verfahren zur Verfahren zur Isolierung laut einem der Ansprüche 2 oder 3, **dadurch gekennzeichnet, dass** das Isolierungsmedium das Davet Medium ist, bestehend aus :
- Ca (NO₃)₂ : 1g,
- CaCl₂, 2H₂O : 1g,
- KNO₃ : 250 mg,
- MgSO₄, 7H₂O 250 mg,
- KH₂PO₄ : 125 mg,
- Saccharose : 2g
- Zitronensäure : 50 mg,
- Agar : 25 g,
- Destilliertes Wasser: 1000 ml;
dann wird das Medium im Autoklaven hingestellt, der pH-Wert wird aud 4,5 mit 1 N HCl eingestellt, und dann Zusatz im warmen Medium des Streptomycinsulfats (30 mg), Vinclozolin (2,5 mg) und Allylalkohol (0,5ml).

6. Verfahren zur Isolierung laut einem der Ansprüche 4 oder 5, **dadurch gekennzeichnet, dass** das Isolierungsmedium das TSM Medium von Elad *et al.* ist, bestehend aus :
- MgSO₄, 7H₂O : 200 mg,
- KH₂PO₄ : 900 mg,
- KCl : 150 mg,
- NH₄NO₃ : 1 g,
- Glukose : 3g,
- Chloramphenicol 250 mg,
- Fenaminosulf : 300 mg,
- Quintozen : 200 mg,
- Bengalrosa : 150 mg,
- Agar : 20 g,
Das Medium wird dann autoklaviert und 200 ml V-8 werden hinzugesetzt, da der pH-Wert zwischen 3,8 und 4 sein soll; solange das Milieu noch warm ist, werden Neomycinsulfat (100 mg), Bacitracin (100 mg), Penicillin G (100 mg), Chloroned (100 mg), Nystatin (20 mg) Chlortetracyclin HCl (25 mg) und Natriumpropionat (500 mg) hinzugefügt.

7. Verfahren zur Isolierung laut einem der Ansprüche 2 oder 3, **dadurch gekennzeichnet, dass** das Isolierungsmedium das Papavizas TME ist, bestehend aus :
- Glukose : 1g,
- Agar : 20 g,
- Destilliertes Wasser: 800 ml,
Das Medium wird dann autoklaviert und 200 ml V-8 werden hinzugesetzt, da der pH-Wert zwischen 3,8 und 4 sein soll; solange das Milieu noch warm ist, werden Neomycinsulfat (100 mg), Bacitracin (100 mg), Penicillin G (100 mg), Chloroned (100 mg), Nystatin (20 mg) Chlortetracyclin HCl (25 mg) und Natriumpropionat (500 mg) hinzugefügt.

8. Verfahren zur Gewinnung eines *Trichoderma atroviride AGR2* enthaltenden Produkts, das nach dem Verfahren laut einem der Ansprüche 2 bis 7 erhalten wurde, **dadurch gekennzeichnet, dass** es aus verschiedenen Phasen besteht:
a. Erzeugung des *Trichoderma atroviride AGR2* Stammes in Petrischalen, die entweder den Sabouraud-Dextrose-Agar oder das Malz-Agar Nährmedium enthalten, enthaltend Chloramphenicol,
b. Isolierung der *Trichoderma atroviride AGR2* Stämme, die in der Form von Sporen sind,
c. Impfen der sterilen Rübenschnitzeln, Kartoffelpülpen, Hafer-, Mais- oder Gerstenflocken, Malz-Agar- oder PDA Nährmedien, mit dem Pilzstamm,
d. Extrudieren des gesamten Substrats, um eine höhere Sporenkonzentration zu erhalten
e. Zusatz von Calciumchlorid.
f. Inkubation in nichtsteriler Kammer für einen Zeitraum von 4 bis 6 Tagen,
g. Trocknen und Mikronisieren in der Form eines Pulvers.

9. Verwendung des *Trichoderma atroviride AGR2* Stammes laut dem "1.
Anspruch" oder durch Anspruch 2 bis 8 erhältlich im Kampf gegen Pflanzenkrankheiten.

10. Verwendung des *Trichoderma atroviride AGR2* Stammes laut dem "1.
Anspruch" oder der durch Anspruch 2 bis 8 erhalten wurde, als Pflanzenschutzmittel durch Sprühen oder durch Tünchen oder durch Wurzeltünchen.

11. Verwendung des *Trichoderma atroviride AGR2* Stammes laut dem "1.
Anspruch" oder der durch Anspruch 2 bis 8 erhalten wurde, als Pflanzenschutzmittel, das nur aus diesem Mikroorganismus besteht.

12. Verwendung des *Trichoderma atroviride AGR2* Stammes laut dem "1.
Anspruch" oder der durch das Verfahren laut einem der Ansprüche 2 bis 8 erhalten wurde, als Pflanzenschutzmittel in Verbindung mit anderen chemischen Fungiziden.

13. Verwendung des *Trichoderma atroviride AGR2* Stammes laut dem "1.
Anspruch" oder der durch das Verfahren laut einem der Ansprüche 2 bis 8 erhalten wurde, als ein Mittel, um gegen Esca , Eutypiose, Black-deadarm, Schwarzfleckenkrankheit und Sclerotinia zu kämpfen.

14. Verwendung des *Trichoderma atroviride AGR2* Stammes laut dem "1.
Anspruch" oder der durch das Verfahren laut einem der Ansprüche 2 bis 8 erhalten wurde, als Pflanzenschutzmittel, das gegen pathogene Pilze wie *Eutypa lata, Phaeomoniella chlamydospora, Phaeomoniella aleophilum, Fomitiporia punctata, Stereum hirsutum, Botryosphaeria dothidea* und *stevensü, Phomopsis viticola, Botrytis cinerea, Sclerotinia minor,*
*sclerotiorum* und *cepivorum, Microdocchium nivale, Armillaria Mella , Phomopsis helianthi* wirksam ist.

15. Verwendung des *Trichoderma atroviride AGR2* Stammes laut dem "1.
Anspruch" oder der durch das Verfahren laut einem der Ansprüche 2 bis 8 erhalten wurde, als Produkt für die Stimulierung der Wurzen, der Pflanzenkeimung und des Pflanzenwachstums, insbesondere für Salate.

16. Verwendung des *Trichoderma atroviride AGR2* Stammes laut dem "1.
Anspruch" oder der durch das Verfahren laut einem der Ansprüche 2 bis 8 erhalten wurde, als Organismus dazu fähig, den Holz in die ersten Zentimetern zu kolonisieren.

## Claims

1. *Trichoderma atroviride* strain **characterized in that** it is the *Trichoderma atroviride AGR2* strain registered in the National Collection of Cultures of Microorganisms (Collection Nationale de Cultures de Micro-organismes CNCM) of the Pasteur Institut under the number I- 2738.

2. A method of isolating the *Trichoderma atroviride AGR2* strain according to claim 1, **characterized in that** it comprises the following steps :
a. sampling of the *Trichoderma atroviride AGR2* strain on a soil sample,
b. sprinkling and dispersion of the sample in step above in a petri dish at 37-40 °C containing an isolation medium,
c. gentle agitation until solidification of the medium,
d. incubation for several days,
e. examination, enumeration and isolation of colonies of *Trichoderma atroviride AGR2.*

3. A method of isolating the *Trichoderma atroviride AGR2* strain according to claim1, **characterized in that** it comprises the following steps :
a. sampling of a soil sample composed of organic materials,
b. suspension of the very sample (10g) in 90 ml of sterile water with a drop of Tween,
c. stirring for 30 minutes,
d. sampling of 10 ml of this solution and then serial dilutions up to 10⁻⁶,
e. pouring of 10 ml of each solution in 90 ml of isolation medium, maintained liquid in hot-water bath at 37-40 ° C,
f. homogenization and distribution in Petri dishes,
g. incubation for 3 to 5 days,
h. examination, enumeration and isolation of colonies of *Trichoderma atroviride AGR2.*

4. A method of selection of the *Trichoderma atroviride* strain obtained according to one of claims 2 or 3, **characterized in that** it comprises the following steps:
a. transplanting of isolated colonies on PDA medium, and exposure to light for 4 to 5 days
b. sampling of green spores,
c. suspension of the spores in sterile water,
d. selection of the strongest colony,
e. transplanting in a petri dish,
f. confrontation test with a strain of *Botrytis cinerea.*

5. A method of isolation according to one of claims 2 or 3, **characterized in that** the isolation medium is the Davet medium, consisting of:
- Ca (NO₃)₂ : 1g,
- CaCl₂, 2H₂O : 1g,
- KNO₃ : 250 mg,
- MgSO₄, 7H₂O 250 mg,
- KH₂PO₄ : 125 mg,
- Sucrose : 2g,
- Citric acid : 50 mg,
- Agar : 25 g,
- Distilled water : 1000 ml,
the medium is then autoclaved, with pH adjustment to 4.5 with 1 N HCl ; and then the lightly warm medium is added streptomycin sulfate (30 mg), vinclozolin (2.5 mg), and allyl alcohol (0.5 ml).

6. A method of isolation according to one of claims 4 or 5, **characterized in that** the isolation medium is the TSM Elad *et al* medium, consisting of:
- MgSO₄, 7H₂O : 200 mg,
- KH₂PO₄ : 900 mg,
- KCl : 150 mg,
- NH₄NO₃: 1 g,
- Glucose : 3g,
- Chloramphenicol : 250 mg,
- Fenaminosulf : 300 mg,
- Quintozene : 200 mg,
- Rose bengal : 150 mg,
- Agar : 20 g,
the medium is then autoclaved, and 200 ml of V-8 are added, the pH should be between 3,8 and 4; when it is still lightly warm, the medium is added neomycin sulfate (100 mg), bacitracin (100 mg), penicillin G (100 mg), chloroned (100 mg), nystatin (20 mg ), HCL chlortetracyclin (25 mg), and sodium propionate (500 mg).

7. A method of isolation according to one of claims 2 or 3, **characterized in that** the insolation medium is the TME Papavizas medium, consisting of:
- Glucose : 1 g,
- Agar: 20 g,
- Distilled water: 800 ml,
the medium is then autoclaved, and 200 ml of V-8 are added, the pH should be between 3,8 and 4; when it is still lightly warm, the medium is added neomycin sulfate (100 mg), bacitracin (100 mg), penicillin G (100 mg), chloroned (100 mg), nystatin (20 mg), HCL chlortetracyclin (25 mg), and sodium propionate (500 mg).

8. A method for obtaining a product containing *Trichoderma atroviride AGR2* obtained according to the process of one of claims 2 to 7, **characterized in that** it consists of different stages:
a. Production of *Trichoderma atroviride AGR2* strain performed on Petri dishes containing a Sabouraud culture medium or malt-agar medium, containing chloramphenicol.
b. Isolation of strains of *Trichoderma atroviride AGR2,* under the spores form,
c. inoculation of the strain of the fungus on sterile beet pulp, or potato pup, oat flakes, barley flakes or cornflakes, malt -agar or PDA media,
d. extrusion of the entire substrate to obtain a high concentration of spores,
e. addition of calcium chloride.
f. incubation in non-sterile room for 4 to 6 days,
g. drying and micronisation under powder form.

9. Use of *Trichoderma atroviride AGR2* strain according to claim 1 or obtained according to claim 2-8 in the control of plant diseases.

10. Use of *Trichoderma atroviride AGR2* strain according to claim 1 or obtained according to claim 2 to 8, as plant-protection product by spraying or brushing, or by root brushing.

11. Use of *Trichoderma atroviride AGR2* strain according to claim 1 or obtained according to claim 2 to 8, as plant-protection product consisting of this only microorganism.

12. Use of *Trichoderma atroviride AGR2* strain according to claim 1 or obtained according to the method of one of claims 2 to 8, as plant-protection product in association with other chemical fungicides.

13. Use of *Trichoderma atroviride AGR2* strain according to claim 1 or obtained according to the method of one of claims 2 to 8, as a mean to fight against Esca disease, Eutypiose, dead arm disease and Sclerotinia disease.

14. Application of *Trichoderma atroviride AGR2* strain according to claim 1 or obtained according to the method of one of claims 2 to 8, as plant-protection product acting against pathogenic fungi such as *Eutypa lata, Phaeomoniella chlamydospora, Phaeomoniella aleophilum, Fomitiporia punctata, Stereum hirsutum, Botryosphaeria dothidea* and *stevensii, Phomopsis viticola, Botrytis cinerea, Sclerotinia minor, sclerotiorum* and *cepivorum, Microdocchium nivale, Armillaria Mella , Phomopsis helianthi.*

15. Use of *Trichoderma atroviride AGR2* strain according to claim 1 or obtained according to the method of one of claims 2 to 8, as a product intended for root stimulation, plant germination and plant growth, especially salads.

16. Use of *Trichoderma atroviride AGR2* strain according to claim 1 or obtained according to the method of one of claims 2 to 8, as organism able to colonize the wood on the first few centimeters.
